# EUROPÄISCHE PATENTANMELDUNG

(11) **EP 0 834 309 A2**
(43) Veröffentlichungstag der Anmeldung: **08.04.1998**
(21) Anmeldenummer: 97116219.3
(22) Anmeldetag: 18.09.1997
(51) Int. Cl.: A61K 9/127

(54) **Verwendung einer Liposomenlösung zur Verstärkung der Wirksamkeit und/oder Verminderung der Toxizität von Arzneimitteln**

(30) Priorität: 27.09.1996 DE 19639811
(71) Anmelder: Mesmer, Artur Herzog, Dr., 65555 Limburg (DE)
(72) Erfinder: Mesmer, Artur Herzog, Dr., 65555 Limburg (DE); Scheller, Albert, Dr., 70806 Kornwesthof (DE); Temirow, Juri Pawlowytsch, Dr., 310 070 Charkov (UA); Krassnopolski, Juri Mychajlowytsch, Dr., 310 070 Charkov (UA); Stefanow, Olexandr Viktorowytsch, Dr., Kiew (UA)
(74) Vertreter: Weber, Dieter, Dr.

(57) **Zusammenfassung**

Die Erfindung betrifft die Verwendung einer durch Homogenisieren einer Phosphatidylcholinemulsion in Wasser unter erhöhtem Druck, erhöhter Temperatur und Zusatz von Lactose hergestellten Liposomenlösung zur Verstärkung der Wirksamkeit und/oder Verminderung der Toxizität von Arzneimitteln.

## Beschreibung

Die therapeutische, diagnostische oder prophylaktische Verabreichung von Arzneimitteln mit Einzelwirkstoffen oder Wirkstoffkombinationen ist häufig aufgrund ihrer Toxizität oder Nebenwirkungen auf bestimmte Applikationsdosen beschränkt. Die maximal applizierbare Dosis bestimmt sich hierbei in der Regel aus den Eigenschaften des Arzneimittels selbst, dem Körpergewicht des Patienten und der Art, dem Zeitraum und dem Zweck der Applikation. So ist beispielsweise die maximale Applikationsdosis von Chemotherapeutika in der Onkologie aufgrund ihrer starken zytotoxischen Wirkung auf gesunde Zellen stark beschränkt. In der Regel werden hier geringe Dosen der Chemotherapeutika über einen langen Zeitraum durch Infusion appliziert.

Ein weiterer Nachteil vieler Arzneimittel, der eine Applikation über einen längeren Zeitraum, beispielsweise durch Langzeitinfusion, erforderlich macht, liegt in deren geringen Halbwertszelten im Organismus. Häufig entwickeln solche Arzneimittel ihre Wirkung innerhalb eines kurzen Zeitraumes oder werden vom Organismus schnell abgebaut. Oft ist jedoch eine geringe Wirkstoffkonzentration über einen längeren Zeitraum erforderlich oder erwünscht, wenn die Applikation einer hohen Dosis in einem kurzen Zeitraum beispielsweise mit einem starken toxischen Effekt oder erheblichen Nebenwirkungen verbunden ist.

Ein weiterer Nachteil vieler Arzneimittel, der die Applikation hoher Dosen erst erforderlich macht, ist, daß sie nicht vollständig oder nur zu einem geringen Teil an den Wirkort transportiert werden. So verteilen sich beispielsweise intravenös applizierte Arzneimittel in der gesamten Blutbahn und werden auch in den Bereichen des Organismus aktiv, wo dies nicht erforderlich ist. Die unspezifische Wirkung vieler Arzneimittel kann zu unerwünschten Nebenwirkungen bis hin zu Schädigungen gesunder Teile des Organismus führen.

Aus dem Stand der Technik ist bekannt, Phosphatidylcholin in Verbindung mit verschiedenen pharmakologischen Wirkstoffen oder als Einzelpräparat zur Prophylaxe oder Therapie von Krankheiten einzusetzen. Phosphatidylcholin gehört zu einer Gruppe natürlich vorkommender Phospholipide, die auch als Lecithine bezeichnet werden. Natürliche Lecithine lassen sich sowohl aus Pflanzen (Lecithinum vegetabile), insbesondere aus Sojabohnen, Raps, Erdnüssen oder Weizenkeimen, sowie als tierisches Lecithin aus Eigelb (Lecithinum ex ovo) gewinnen. Aus Eigelb gewonnenes Lecithin enthält als Hauptbestandteil Phosphatidylcholin (in einer Menge von etwa 80 %) und als Nebenbestandteile unter anderem Stearinsäure und Ölsäure. Dieses tierisch gewonnene Phosphatidylcholin wird auch als Lipin bezeichnet.

Pharmazeutisch verwendet wird derzeit hauptsächlich Phosphatidylcholin aus Sojabohnenlecithin, wobei letzteres ein Nebenprodukt bei der Ölgewinnung aus Sojabohnen ist. Das sogenannte Rohlecithin wird aus dem rohgepreßten Öl gewonnen, indem dieses mit Wasser versetzt wird, woraufhin sich die Phospholipide in der Grenzschicht zwischen Öl und Wasser absetzen. Anschließend werden diese in einem Separator abzentrifugiert. Aus dem als Naßschlamm bezeichneten Produkt erhält man durch Abdampfen des Wassers ein öliges Produkt, das anschließend je nach Anwendung unterschiedlichen Reinigungsprozeduren unterzogen wird.

Verwendet werden Phosphatidylcholine bzw. Lecithine hauptsächlich als Emulgatoren und Suspensionsstabilisatoren in der pharmazeutischen Technologie und Lebensmittelindustrie. Therapeutisch finden Lecithine Anwendung als Venenmittel oder als Lipidsenker, da man herausgefunden hat, daß sie zur Reduktion der Serumcholesterinkonzentration beitragen können. Als Bestandteil von Kombinationspräparaten werden sie zur Behandlung von Hyperlipoproteinämien eingesetzt. Weitere Anwendungsgebiete sind die Arterioskleroseprophylaxe, akute und chronische Hepatopatien und leberabhängige Gestosen. Die Wirksamkeit bei den bekannten Anwendungsgebieten ist jedoch strittig.

Das aus Eigelb gewonnene tierische Phosphatidylcholin, Lipin, wird bisher als Einzelwirkstoff in der Pulmonologie eingesetzt. Hier wird es zur Behandlung und Prophylaxe verschiedener Lungen- und Atemwegserkrankungen verwendet, wie beispielsweise bei der Behandlung des Syndroms akuter und chronischer Ateminsuffizienz, wobei man annimmt, daß es eine gestörte phospholipidische Zusammensetzung des Lungensurfaktants normalisiert. Klinische Untersuchungen lassen darauf schließen, daß Lipin die Ventilationsfunktion der Lunge normalisiert, die Diffusion des Sauerstoffes aus der Alveolarluft in das Blut und aus diesem in das Gewebe begünstigt, arterielle Hypoxämie abbaut, die Liquidierung der Lactazidose beschleunigt und die Oxidation von Lipiden durch freie Radikale vermindert. Weiterhin wurde gezeigt, daß Lipin bronchiolytisch und mukolytisch wirkt und zur Erhöhung der unspezifischen Immunität des Organismus beiträgt. Dementsprechend findet Lipin bei erblich bedingter Lungenmukoviszidose und erworbenen Lungenerkrankungen Anwendung, sowie bei chronischer Pneumonie, akuter und chronischer, obstruktiver und nichtobstruktiver Bronchitis, Bronchialasthma, bronchiektatischer Krankheit, Lungenabszessen und verschiedenen Formen der Lungenhypoplasie.

Bei Anwendung in der Pulmonologie wird gelöstes bzw. suspendierte Lipin inhaliert oder intravenös verabreicht. Die Toxizität von Lipin beträgt für Warmblüter 9,0 bis 12,0 g/kg der Körpermasse. Bei therapeutischer Dosierung ist das Präparat für den Menschen gut verträglich. Auch ist die Verwendung von Lipin als antihypoxisches Mittel bekannt. Eine Potenzierung von Arzneimitteln durch Phosphatidylcholin oder ein Einfluß desselben auf die Toxizität von Arzneimitteln ist in der Literatur nicht beschrieben.

Weiterhin ist bekannt, Pflanzenlecithin, bevorzugt Sojalecithin, als Pulver oder als Emulsion in wäßrigem Medium mit anderen pharmazeutischen Wirkstoffen zu kombinieren. Die Aufgabe und Wirkung des Lecithins in diesen Kombinationspräparaten liegt im wesentlichen in der Stabilisierung der zu verabreichenden Hauptwirkstoffe. Eine signifikante Beeinflussung der kombinierten Wirkstoffe durch das Pflanzenlecithin wurde dabei nicht beobachtet.

Aufgabe der Erfindung war es, die Wirksamkeit von Arzneimitteln gegenüber dem Stand der Technik zu verstärken und/oder deren Toxizität zu vermindern.

Diese Aufgabe wird dadurch gelöst, daß die Arzneimittel zusammen mit einer durch Homogenisieren einer Phosphatidylcholinemulsion in Wasser unter erhöhtem Druck, erhöhter Temperatur und Zusatz von Lactose hergestellten Liposomenlösung angewendet werden. Zweckmäßigerweise wird dafür die Liposomenlösung in einem Mischungsverhältnis von Phosphatidylcholin zu Arzneimittel von 100 : 1 bis 1 : 1, bevorzugt 60 : 1 bis 20 : 1, besonders bevorzugt 50 : 1 bis 30 : 1, bezogen auf die Trockensubstanzen, eingesetzt. Als besonders günstig hat es sich erwiesen, wenn die Homogenisierung der Phosphatidylcholinemulsion unter einem Druck von 5,9 x 10⁷ bis 6,3 x 10⁷ Pa, bei einer Temperatur von 40 bis 45 °C und unter Zusatz von Lactose in einer Menge von 1,8 bis 2,2 Gew.% erfolgt und zweckmäßig zusätzlich die Herstellungsstufen der Entkeimungsfiltration, Abfüllung und lyophiler Austrocknung umfaßt.

Besonders vorteilhaft ist die erfindungsgemäße Verwendung einer durch Homogenisieren einer Phosphatidylcholinemulsion hergestellten Liposomenlösung, die tierisches, vorzugsweise aus Eidotter gewonnenes, Phosphatidylcholin enthält. Dieses tierische Phosphatidylcholin hat sich gegenüber pflanzlichem Phosphatidylcholin bei der erfindungsgemäßen Anwendung erheblich wirksamer gezeigt.

Im folgenden wird der Begriff "Liposomenlösung" für eine entsprechend dem Vorgenannten und den Patentansprüchen 1 bis 4 durch Homogenisieren einer Phosphatidylcholinemulsion hergestellten Liposomenlösung verwendet.

Überraschenderweise hat sich gezeigt, daß eine Vielzahl von Arzneimitteln bzw. Wirkstoffen für eine große Anzahl therapeutischer Anwendungen gegenüber den aus dem Stand der Technik bekannten Wirkungen der freien oder kombinierten Formen dieser Arzneimittel besonders vorteilhafte prophylaktische, diagnostische oder therapeutische Eigenschaften aufweisen, wenn sie mit einer Liposomenlösung gemischt oder zusammen mit dieser verabreicht werden, die nach dem obengenannten Verfahren hergestellt wurde. Eine Verabreichung von Arzneimitteln zusammen mit einer Liposomenlösung kann auch durch getrennte Verabreichung innerhalb eines kurzen Zeitraumes erfolgen, z. B. durch aufeinanderfolgende orale Aufnahme oder getrennte Gabe von Arzneimittel und Liposomenlösung durch nacheinander gespritzte Injektionen. Jede andere getrennte Verabreichung innerhalb eines kurzen Zeitraumes ist denkbar.

Die mit der speziellen Liposomenlösung kombinierten Arzneimittel weisen eine erheblich geringere Toxizität als die freie Form des entsprechenden Arzneimittels auf. Ein besonderer Vorteil dieser antitoxischen Wirkung der speziellen Liposomenlösung liegt darin, daß die Kombination mit Arzneimitteln es erst ermöglicht, höhere als die bislang möglichen Dosen verschiedener Arzneimittel einzusetzen, ohne daß diese ihre toxische Wirkung entfalten können. Eine Herabsetzung der Toxizität durch Kombination mit der speziellen Liposomenlösung wurde beispielsweise mit verschiedenen Antikonvulsiva, Antidepressiva, Antiepileptika, zentralen Analgetika, Antihistaminika, Parasympatholytika sowie mit Nitraten, Betablockern, Thrombolytika, Vitaminen, Mineralstoffen, Antiaggregationsmitteln und verschiedenen Zytostatika, wie beispielsweise einigen Anthrazyklinantibiotika, beobachtet.

Die toxische Dosis vieler Arzneimittel liegt bei gemeinsamer Applikation mit der speziellen Liposomenlösung in einem Mischungsverhältnis von 1 : 1 bis 1 : 100 bei bis zu 300 % der toxischen Dosis des Arzneimittels in der freien Form.

Ein weiterer Vorteil der Applikation des Arzneimittels zusammen mit der Liposomenlösung besteht darin, daß die Halbwertszeit vieler Arzneimittel erheblich erhöht wird. Der Abbau der Arzneimittel im Organismus wird deutlich verzögert, so daß es wesentlich länger im Blutkreislauf nachgewiesen werden kann als die freie Form des gleichen Stoffes. Dies ermöglicht einerseits, die zu applizierende Dosis eines Arzneimittels herabzusetzen, da der Wirkstoff von körpereigenen Abbaumechanismen langsamer beziehungsweise in geringerem Maße abgebaut wird, und andererseits können dem Patienten Dauerapplikationen, wie Langzeitinfusionen, verkürzt oder erspart werden.

Es hat sich gezeigt, daß bei der erfindungsgemäßen Verwendung der speziellen Liposomenlösung eine Depotwirkung erzielt werden kann, bei der die Arzneimittel über einen längeren Zeitraum als die freie Form aktiv sind und ihre Wirkung über diesen Zeitraum in geringeren Dosen kontinuierlich freisetzen. Schädigungen des Organismus aufgrund hoher Wirkstoffkonzentrationen und Nebenwirkungen können somit stark vermindert oder gar vermieden werden.

Ein weiterer Vorteil der erfindungsgemäßen Verwendung der speziell hergestellten Liposomenlösung in Kombination mit verschiedenen Arzneimitteln liegt in einem erheblich verbesserten Transport dieser Arzneimittel an den gewünschten Wirkort. Beispielsweise verbessert die gemeinsame Applikation vieler Arzneimittel zusammen mit der Liposomenlösung den Transport dieser Arzneimittel durch die Membran in das Innere von Zellen. Phosphatidylcholin hat hierbei eine Trägerfunktion, durch die die Translokation des Arzneimittels durch die Zellmembran erleichtert wird.

Weiterhin wurde überraschenderweise herausgefunden, daß bei der erfindungsgemäßen Verwendung der speziellen Liposomenlösung wesentlich größere Mengen des Arzneimittels die Bluthirnschranke überwinden können als dies bei Arzneimitteln in der freien Form der Fall ist. Durch die Kombination verschiedener Arzneimittel mit der Liposomenlösung ist es somit möglich, Behandlungen des zentralen Nervensystems, wie die Chemotherapie von Gehirntumoren oder ähnlichem, durch vereinfachte Applikation und mit geringeren Wirkstoffmengen durchzuführen.

Überraschenderweise hat sich gezeigt, daß zusammen mit der Liposomenlösung intraarteriell applizierte Zytostatika im Tumorgewebe akkumuliert werden und in den ersten vier Stunden nach der Applikation 1,5-bis 2,5mal so viele Zellkontakte nachweisbar ist wie mit der freien Form des Zytostatikums. Die intravenöse Gabe von Zytostatika zusammen mit Lipin führt zur Zerstörung cancerogener Komplexe von hochdifferenzierten Adenoscarcinomen und zur Zerstörung der Tumorzellen. Die Zellkontakte der Krebszellen werden aufgelöst und die Zellstrukturen degradiert.

Die erfindungsgemäße Verwendung der speziellen Liposomenlösung in Kombination mit entsprechenden Arzneimitteln ist zur Verstärkung der Wirksamkeit, Verminderung der Toxizität, zum verbesserten Transport zum oder zur Akkumulation am Wirkort und/oder zur verbesserten Dosierung im Vergleich zum freien Arzneimittel bei folgenden Indikationen angezeigt:

Kreislauf- und Gefäßerkrankungen jeder Genese, Herzkrankheiten, Stoffwechselstörungen bei Durchführung akuter thrombolytischer Therapien, vor, während und nach der Thrombolyse, Störungen der immunologischen Abwehrfunktion im Bereich der Naturalkillerzellen, Monozyten, Makrophagen, Granulozyten, T- und B-Lymphozyten und Plasmazellen, Störungen der Komplementfaktoren und der Antikörpersynthese, Störungen der Lymphokinbiosynthese bei Makrophagen und anderen Zellen, Kardiomyopathien jeglicher Genese auch in Verbindung mit anderen Therapien, alle Formen von Koronarleiden, Angina pectoris, Myokardie, Myokardinfarktprophylaxe, Herzinfarkt, Skelettmuskelstörungen, alle Formen von Blutzellerkrankungen, Anämien, Leukopenien, Thrombozytopenien, Plasmamangel, Krebserkrankungen jeder Genese, Metastasen, maligne Primärtumoren, Hepatopathien, insbesondere akute und chronische sowie infektiöse und mechanisch toxische Hepatitis, Viruserkrankungen, bakterielle Erkrankungen, durch Protozoen bedingte Hepatitiden, Fettleber, Fettzirrhose, Leberzirrhose jeder Genese, Lipidüberoxidation, lipidoxidativem Streß im Rahmen der Anwendung einer Hyperthermietherapie, Radiotherapie, Chemotherapie oder Therapie mit aktiviertem Sauerstoff, Proliferationsstörungen und Differenzierungsstörungen von Epithel-, Endothel- und Schleimhautgeweben, vorzeitigem Altern, Altersverschleiß aller Gewebe, Organe, Gefäße oder des Herzkreislaufsystems.

Die gemeinsame Verabreichung der erfindungsgemäßen Liposomenlösung zusammen mit Kardioprotektiva und Zytostatika hat sich besonders zur Behandlung von Herzkreislauferkrankungen und Herzsarkomen sowie zur Vorbeugung der Metastasenentstehung als vorteilhaft erwiesen. Die erfindungsgemäße Lipidemulsion kann in Mengen verabreicht werden, die 10 bis 30 mg Phosphatidylcholin pro Kilogramm Körpergewicht entsprechen. Als Kardioprotektiva sind hierbei folgende Substanzen besonders geeignet:

Aminoglykoside, Propranol, Verapamil, Pindolol, Klophelin, Korinfar, Korduron, Sotalol, Magnesium, Sprironolakton, Triamteren, Indometazin, Ibuprofen, Acetylsalicylsäure, Heparin, EDTA, Nitrate, Alpha- und Betablocker, Niphedipin, Plasmin, Plasminogen, Streptokinase, Streptodekase, Antioxidantien, Vitamine, Mineralien und Spurenelemente.

Die gemeinsame Verabreichung von Streptokinase, Betablockern und der erfindungsgemäßen Liposomenlösung beugt Manifestierung von Herzschmerzsyndromen vor, normalisiert Herzrhythmusstörungen, reduziert die Gefahr der Entstehung von Herzinfarkten und beugt diesen vor und revidiert den Verlauf und die Symptome instabiler Angina Pectoris.

Die Applikation der erfindungsgemäß verwendeten Liposomenlösung zusammen mit entsprechenden Arzneimitteln kann intravenös als Infusion, intramuskulär, intrakutan, subkutan, oral, nasal, buccal, sublingual, per Inhalation, vaginal oder rektal erfolgen. Als besonders geeignet hat sich die intravenöse Injektion oder Infusion herausgestellt, wobei die Infusion, insbesondere bei hoher Dosierung, über einen längeren Zeitraum hinweg zweckmäßig ist. Als Grundlage für die Zubereitung von Infusionslösungen mit Phosphatidylcholin eigenen sich physiologische Kochsalzlösungen, deren Aufbewahrung lichtgeschützt erfolgen sollte. Die orale Applikation ist in Form von Tabletten, Kapseln, Pulver oder Tropfen möglich. Die intravenöse Applikation ist der oral enteralen Aufnahme vorzuziehen, da bei manchen Arzneimitteln durch oxidative Reaktionen eine Verminderung der Wirkung eintreten kann. Gleiches gilt für die Applikation durch Inhalation. Sofern die medizinischen Voraussetzungen gegeben sind, ist eine intratumorale oder auch intraperitoneale Applikation zweckmäßig. Für die äußerliche Applikation eignen sich Salben, Sprays oder Lösungen.

Die therapeutische Dosierung der Liposomenlösung, die sich im Tierversuch und beim Menschen als unbedenklich und zweckmäßig erwiesen hat, liegt in einem Bereich von 0 bis 1 g/kg Körpergewicht Phosphatidylcholin, bezogen auf den Feststoff. In akuten Fällen können auch höhere Dosen verwendet werden, wobei zu berücksichtigen ist, daß die toxische Dosis für Tier und Mensch etwa 9 bis 12 g/kg Körpergewicht Phosphatidylcholin beträgt.

Die erfindungsgemäße Verwendung der speziellen Liposomenlösung ist zur Kombination mit den folgenden Arzneimitteln oder Wirkstoffen besonders geeignet:

Nifedipin, Plasmin, Plasminogen, Betablocker, Acetylsalicylsäure, Prednisolon, Phenobarbital, Paracetamol-Panadol, EDTA, Heparin, Chinin, Atropin, Theophylin, Corticosteroide jeder Art, Antikonvulsiva jeder Art, Digitalis, Verapamil, Antidepressiva jeder Art, Antibiotika jeder Art, Antiepileptika, zentrale Analgetika, Parasympatholytika, Antiparkinsonmittel, Antihistaminika, trizyklische Antidepressiva, Diuretika, Antidiabetika, Muskelrelaxantien aller Art, Aminoglutethimid, Dexamethason, Digitoxin, Asparaginase, Medroxyprogesteronacetat, Megestrolacetat, ACE-Hemmer, Azathioprin, Allopurinol, Etoposid, Cyclophosphamid, Ciclosporin A, Lipopolysaccharide jeder Art, Doxorubicin, Daunorubicin, Dichlofenack, Dipetiazem, Famotidin, Fluconazol, Halothan, Clipizid, Griseofulvin, Indometacin, Inthraconazol, Josamycin, Ketoconazol, Levonantrodol, Mesna, Melphalan, Antifolate, Methotrexat, Morphine und Opiate jeder Art, Omeprazol, Rifampicin, Sulfadiazin, Pentostatin, Pethidin, Thymidin, Procarbazin, Ranitidin, Hydroxyharnstoff, Tamoxifen, Idarubicin, Amphotericin B, Fotemustin, Chlorambucil, Immunstoffe aller Art, Interleukine aller Art, Immunotoxine, Ricin-Pertusis, Diphterietoxin, Interferone alpha, beta und gamma, Östrogene, Phenazon, Warfarin, Vinonelbin, Navelbin, Selen und selenhaltige Verbindungen, Streptozotocin, Thiole, G-SH-Acetyl-G-SH, Trimethoprim, Sulfadiacin, Vinblastin, Vincristin, Cimetidin, Epirubizin, Idarubizin, Mitoxantron, Estramustinphosphat, Thiotepa, Procarbazin, Nitrosoharnstoffe, CCNU, Me-CCNU, BCNU, ACNU, PCZ, UM-26, VP-16, Hexitolderivate, DAG, DBD, DIAC, Butyl-Scopolamin, Baclofen, Opioide, Dipidolor, Tramadol, Chlormezanon, Codein, Clonazepam, Dehydrocodein, Clonidin, Morphin, Hydromorphin, L-Polamidon, L-Methadon, Fentanyl, Damidronat, Haloperidol, Buskopan, Metamizol, Cytarabin, Dapson, Bleomycin, Acetyldigoxin, Cisplatin, Carboplatin, Platinpräparate und Platinsalze jeder Art, Phenytoin, Sauerstoff, Lachgas, Busulfan, Aminoglukoside, Ifosfamid, Natriumthiosulfat, Carmustin, Polio- und BCSCH-Impfstoffe und Impfstoffe aller Art, Chlorozotocin, Mitomycin C, Superoxidwismutase, Adreamycin, Plasmaproteine, Salicylate, Levamisol, Pyridoxin, Tauromustin, Fluorouracil, Phtoraphur, Calciumfolinat, Cystein, Dacarbacin, Monoaminoxydasehemmstoffe, Hexamethylmelamin, Lomustin, Semustin, Mercaptopurin, Cotrimoxazol, Nukleoside und Nukleosidanaloga jeder Art, Alkohol, Aminophenazon, Antirheumatika, Glafenin, Ketoprofen, Phenylbutazon, Prednison, Probenecid, Mitomycin, Diphosphonate, Calcitonin, Glucagon, Furosemid, Glycylpressin, Mitoxantron, N-Acetylcystein, Ceftacidim, Paclitaxel, Taxol, Chinidin, Ketonazol, Vitamine A, E, B, C und D, Antioxidantien jeder Art, Blutfarbstoffe jeder Art, Procarbazin, Sargromostim, Lithium, Phenytoln, Toremifen, Jomol, Jomotech, Jomoin, Purinantagonisten, Fluorarabinphosphate, Mexicitin, Midazolam, Zuckerlösung, Tetrazepam, Propanadol, Pindolol, Klophelin, Kordaron, Sotalol, Magnesium, Spironolakton, Triamteren, Aminoglycoside, DNA- und RNA-Extrakte aus Stöhrfisch oder Kalbsthymus, Betulinsäure, Akridon- und Akridanonverbindungen, .

Weiter Vorteile, Merkmale und Anwendungsmöglichkeiten werden deutlich anhand der folgenden Beispiele:

### Beispiel 1

### Vergleich der Toxizitäten der Anthracyclin-Antibiotika Doxorubicin und Carminomycin in der freien Form und in Kombination mit Phosphatidylcholin

Im Tierexperiment mit Mäusen wurde die Toxizität verschiedener Konzentrationen der Antibiotika Doxorubicin und Carminomycin in der freien Form und in Kombination mit Phosphatidylchlolin untersucht. Doxorubicin und Carminomycin gehören zu den Anthracyclinen, einer Gruppe von Antibiotika, die wegen ihrer toxischen Eigenschaften nicht zur Behandlung bakterieller Infektionen verwendet werden. Sie haben jedoch Eingang in die Therapie als besonders wichtige Zytostatika gefunden. Ihre zytotoxische Wirkung ist in der S-Phase der betroffenen Zellen am stärksten ausgeprägt und beruht auf der Interkalation des Wirkstoffes in die zelluläre DNA, was zur Hemmung der Nucleinsäuresynthese führt. Der Einsatz der Anthracycline wird allerding durch ihre Kardiotoxizität eingeschränkt. Diese ist mit der applizierten Gesamtdosis korreliert und häufig irreversibel. Vermutlich beruht die herzschädigende Aktivität der Anthracycline im wesentlichen auf der Bildung von Radikalen.

Doxorubicin und Carminomycin wurden jeweils in Lösung mit Phosphatidylcholin in einem Verhältnis von 1 : 40, bezogen auf die Feststoffe, gemischt und in Mäuse injiziert. In Parallelexperimenten wurden Mäuse mit den jeweiligen Anthracyclinen in der freien Form behandelt. Doxorubicin wurde in Dosen von 5, 15, 20, 25 und 30 mg/kg Körpergewicht der jeweiligen Maus und Carminomycin in Dosen von 1, 5, 10 und 15 mg/kg Körpergewicht der jeweiligen Maus appliziert. Nach 25 Tagen wurde die Anzahl der überlebenden Mäuse bestimmt, die mit der freien Form des Antibiotikums bzw. der mit Phosphatidylcholin gemischten Form behandelt worden waren. Tabelle I faßt die Ergebnisse dieses Experiments zusammen. Es konnte gezeigt werden, daß die Toxizität von Doxorubicin bzw. Carminomycin durch das Vermischen bzw. die gleichzeitige Applikation zusammen mit Phosphatidylcholin um ein Vielfaches herabgesetzt werden konnte.

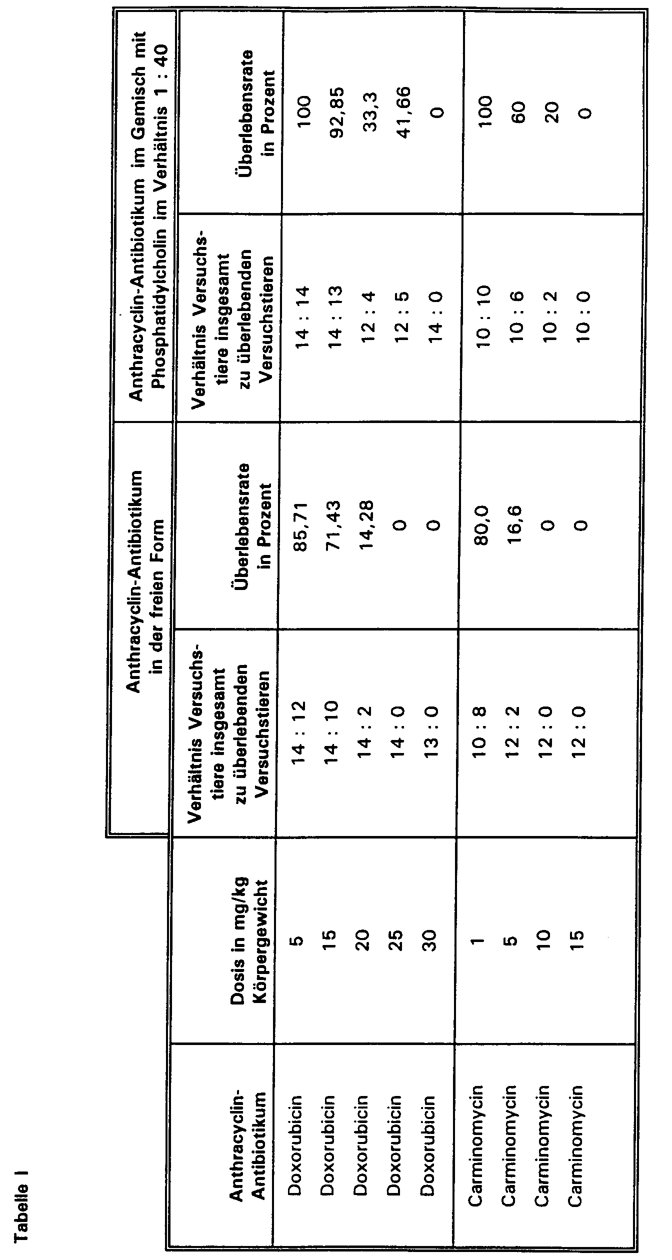

In regelmäßigen Abständen wurden den Versuchstieren Blutproben entnommen und die Konzentrationen der verabareichten Antibiotika im Blutserum bestimmt. Es wurde festgestellt, daß die Konzentrationen von Doxorubicin und Carminomycin im Blutserum der Versuchstiere wesentlich schneller abnahmen, wenn sie in der freien Form injiziert wurden, als dies der Fall war, wenn sie zusammen mit Phosphatidylcholin im Verhältnis 1 : 40 appliziert wurden. Die Konzentrationen der zusammen mit Phosphatidylcholin verabreichten Antibiotika lagen um bis zu 300 % höher als die Konzentrationen der in der freien Form gegebenen Arzneimittel. Es wird angenommen, daß die gleichzeitige Verabreichung von Phosphatidylcholin entweder den Abbau der Antibiota im Blut, deren Freisetzung oder beides verlangsamt. Auch die Herabsetzung der Toxizität der Antibiotika kann durch die Depotwirkung der Liposomenlösung erklärt werden. Die verlangsamte Freisetzung des Wirkstoffes im Blutkreislauf verhindert die Überkonzentration des Antibiotikums während eines kurzen Zeitraums, die letztlich für die toxische Wirkung verantwortlich ist. Neben der Herabsetzung der Toxizität wird durch die Depotwirkung des Phoxphatidylcholins auch eine verlängerte Verfügbarkeit der Antibiotika im Organismus erzielt.

Ein besonders überraschender Vorteil der gemeinsamen Verabreichung von Doxorubicin bzw. Carminomycin mit der speziellen Liposomenlösung war, daß erheblich geringere Konzentrationen dieser beiden Substanzen im Herzmuskel der Versuchstiere nachgewiesen werden konnten, als dies der Fall war, wenn Doxorubicin bzw. Carminomycin in der freien Form appliziert wurde. Auch diese Beobachtung kann die geringere Letalität der Versuchstiere, denen das Präparat zusammen mit Phosphatidylcholin injiziert worden war, erklären. Aufgrund der geringeren Akkumulation der Substanzen im Herzmuskel konnten diese ihre kardiotoxische Aktivität in diesem Organ nicht voll entfalten.

Weiterhin wurden gegenüber den in der freien Form appllzierten Antibiotika erhöhte Konzentrationen von Doxorubicin bzw. Carminomycin, die zusammen mit der Liposomenlösung appliziert wurden, im Gehirn der Versuchstiere nachgewiesen. So konnte gezeigt werden, daß Phosphatidylcholin in der Lage war, einen Teil der Wirkstoffe durch die gematoenzephalitische Barriere zu transportieren und im Gehirn zu speichern. Dadurch ergeben sich neue Möglichkeiten zur effektiveren Behandlung von Gehirnerkrankungen, die zuvor aufwendige und gefährliche Verfahren erforderten.

Ein weiterer Vorteil der Verabreichung von Doxorubicin und Carminomycin zusammen mit Phosphatidylcholin war, daß diese in erheblich geringerem Maße als die freien Formen in der Leber gespeichert wurden. Es ist bekannt, daß die Anlagerung verschiedenster Arzneimittel in der Leber zu starken Schädigungen führen kann. Auch hier bietet Phosphatidylcholin die Möglichkeit, die Gefahr einer Leberschädigung als Nebenwirkung von Medikamenten herabzusetzen.

### Beispiel 2

### Untersuchung der Toxizität von Fluorourazil in der freien Form und in Kombination mit Phosphatidylcholin

Fluorourazil ist ein Antimetabolit und wird aufgrund der Zytotoxizität als Zytostatikum zur Paleativbehandlung von Mamma-, Rektum- und Kolonkarzinomen, ferner zur Therapie von Pankreaskarzinomen, primären Leber-, Ovarial-, Uterus- und Blasenkarzinomen eingesetzt, wenn andere Behandlungsmöglichkeiten erschöpft bzw. nicht indiziert sind.

Fluorourazil wurde, wie die Anthrazyklinantibiotika Doxorubicin und Carminomycin aus Beispiel 1, mit der Liposomenlösung im Verhältnis 1 : 40, bezogen auf den Feststoff Phosphatidylcholin, gemischt und Mäusen in Dosen von 1,5, 2,0, 2,5, 3,0, 3,5 und 4,0 mg/kg Körpergewicht injiziert. Im Parallelexperiment wurden weiteren Versuchstieren die gleichen Dosen Fluorourazil in der freien Form appliziert. Nach 25 Tagen wurde die Anzahl überlebender Versuchstiere bestimmt. Die Ergebnisse des Experimentes sind in Tabelle II wiedergegeben.

**Tabelle II**

| | **Fluorouracil in der freie Form** | | **Fluorouracil im Gemisch mit Phosphatidylcholin im Verhältnis 1 : 40** | |
|---|---|---|---|---|
| **Dosis in mg/kg Körpergewicht** | **Verhältnis Versuchstiere insgesamt zu überlebenden Versuchtstieren** | **Überlebensrate in Prozent** | **Verhältnis Versuchstiere insgesamt zu überlebenden Versuchtstieren** | **Überlebensrate in Prozent** |
| 1,5 | 12 : 9 | 75 | 10 : 10 | 100 |
| 2,0 | 12 : 9 | 75 | 10 : 10 | 100 |
| 2,5 | 12 : 4 | 33 | 12 : 10 | 83 |
| 3,0 | 12 : 0 | 0 | 14 : 7 | 50 |
| 3,5 | 14 : 0 | 0 | 12 : 2 | 16 |
| 4,0 | 10 : 0 | 0 | 10 : 0 | 0 |

Die Ergebnisse dieser Experimente zeigen, daß die Kombination von Fluorourazil mit Phosphatidylcholin eine erheblich geringere Toxizität für die Versuchstiere besaß als die freie Form von Fluorourazil. Ebenso wie zuvor Doxorubicin und Carminomycin aus Beispiel 1 besaß Fluorourazil in diesem Experiment eine erheblich längere Halbwertszeit im Blut des Organismus der Versuchstiere, wenn es in Kombination mit Phosphatidylcholin injiziert wurde, als die freie Form.

Weiterhin wurde festgestellt, daß sich Fluorourazil in Kombination mit Phosphatidylcholin in erheblich geringeren Konzentrationen im Herzmuskel anlagerte als Fluorourazil in der freien Form. Dies ist eine besonders vorteilhafte Eigenschaft, da Fluorourazil, wie auch die Anthrazyklinantibiotika aus Beispiel 1, starke kardiotoxische Aktivität besitzt.

Untersuchungen der Gehirne der Versuchstiere zeigten, daß wesentlich mehr Fluorourazil, das mit der Liposomenlösung zusammen verabreicht wurde, die Bluthirnschranke durchdringen konnte als Fluorourazil in der freien Form. Auch dieses Beispiel zeigt, daß die Verwendung von Phosphatidylcholin in Kombination mit Zytostatika besonders geeignet ist, Wirkstoffe aus dem Blutkreislauf ins Gehirn zu transportieren und dort anzulagern. Daher ist die kombinierte Anwendung der speziellen Liposomenlösung mit Zytostatika besonders zur Therapie von Gehirntumoren und anderen Gehirnerkrankungen geeignet.

### Beispiel 3

### Untersuchung der Toxizität von Cisplatin in der freien Form und in Kombination mit Phosphatidylcholin

Cisplatin ist ein Zytostatikum, welches bei Krebserkrankungen von Lunge, Hoden, Eierstöcken, Gebärmutterhais, Prostata, Gebärmutterschleimhaut, Harnblase, Haut, Bindegewebsgeschwülsten, Krebserkrankungen des Kopf- und Hals-Bereiches, kleinzelligen und nichtkleinzelligen Bronchialkarzinomen, Plattenepithelkarzinomen, Melanomen und Sarkomen eingesetzt wird. Wie auch die Zytostatika aus den Beispielen 1 und 2 besitzt Cisplatin kardiotoxische Aktivität und kann zu vaskulären Ereignissen, wie z. B. Apoplexie, Myokardinfarkt oder peripheren Durchblutungsstörungen, führen.

Cisplatin wurde mit der Liposomenlösung in einem Verhältnis von 1 : 40, bezogen auf die Trockenmassen, gemischt und in Dosen von 5, 10, 15, 20 und 25 mg/kg Körpergewicht in Mäuse injiziert. In Parallelexperimenten wurde Cisplatin in der freien Form in gleichen Dosen appliziert. Nach 25 Tagen wurde die Überlebensrate der Versuchstiere bestimmt. Die Ergebnisse dieser Experimente sind in Tabelle III zusammengefaßt. Wie auch in den Experimenten der Beispiele 1 und 2 hat hier die gleichzeitige Verabreichung von Phosphatidylcholin zusammen mit Cisplatin die Überlebensrate der Versuchstiere erheblich erhöht. Die Experimente zeigen, daß die erfindungsgemäße Verwendung der speziellen Liposomenlösung eine erhebliche Absenkung der Toxizität, eine wesentlich längere Verfügbarkeit des Wirkstoffes im Blutkreislauf und eine Erhöhung der Konzentration des Cisplatins im Gehirn der Versuchstiere zur Folge hatte. Auch Cisplatin läßt sich zweckmäßig zusammen mit Phosphatidylcholin zur Behandlung von Gehirntumoren verabreichen.

**Tabelle III**

| | **Cisplatin in der freien Form** | | **Cisplatin im Gemisch mit Phosphatidylcholin im Verhältnis 1 : 40** | |
|---|---|---|---|---|
| **Dosis Cisplatin in mg/kg Körpergewicht** | **Verhältnis Versuchstiere insgesamt zu überlebenden Versuchtstieren** | **Überlebensrate in Prozent** | **Verhältnis Versuchstiere insgesamt zu überlebenden Versuchtstieren** | **Überlebensrate in Prozent** |
| 5 | 10 : 8 | 80 | 10 : 9 | 90 |
| 10 | 10 : 5 | 50 | 10 : 4 | 40 |
| 15 | 10 : 1 | 10 | 10 : 3 | 30 |
| 20 | 10 : 0 | 0 | 10 : 1 | 10 |
| 25 | 10 : 0 | 0 | 10 : 0 | 0 |

### Beispiel 4

### Chemotherapie von Patienten mit inoperablem Magen- bzw. Kolonkarzinom

Im klinischen Experiment wurden Krebspatienten mit inoperablen Magen- bzw. Kolonkarzinomen entweder auf herkömmliche Weise in erfindungsgemäßer Kombination mit der speziellen Liposomenlösung mit dem Zytostatikum 5-Fluorurazil (5FU) behandelt. Pro Aplikation wurden je nach Körpergewicht des Patienten 500 - 800 mg 5-Fluorurazil verabreicht, jedoch nicht mehr als 7 g über den gesamten Behandlungszeitraum. Der Krankheitsverlauf, d. h. die Entwicklung der Karzinome, wurde röntgenologisch unter Einsatz von Kontrastmittelverstärkung verfolgt. Die Behandlungsergebnisse wurden je nach Erfolg in vier Kategorien eingeteilt, nämlich "volle Regression", d. h. vollständige Rückbildung des Karzinoms, "Teilregression", "stabiler Zustand", d. h. weder Zu- noch Abnahme des Karzinoms, und "Progression des Wachstums", d. h. weiteres Wachstum des Karzinoms. Die Ergebnisse der Unterschungen sind in Tabelle IV wiedergegeben.

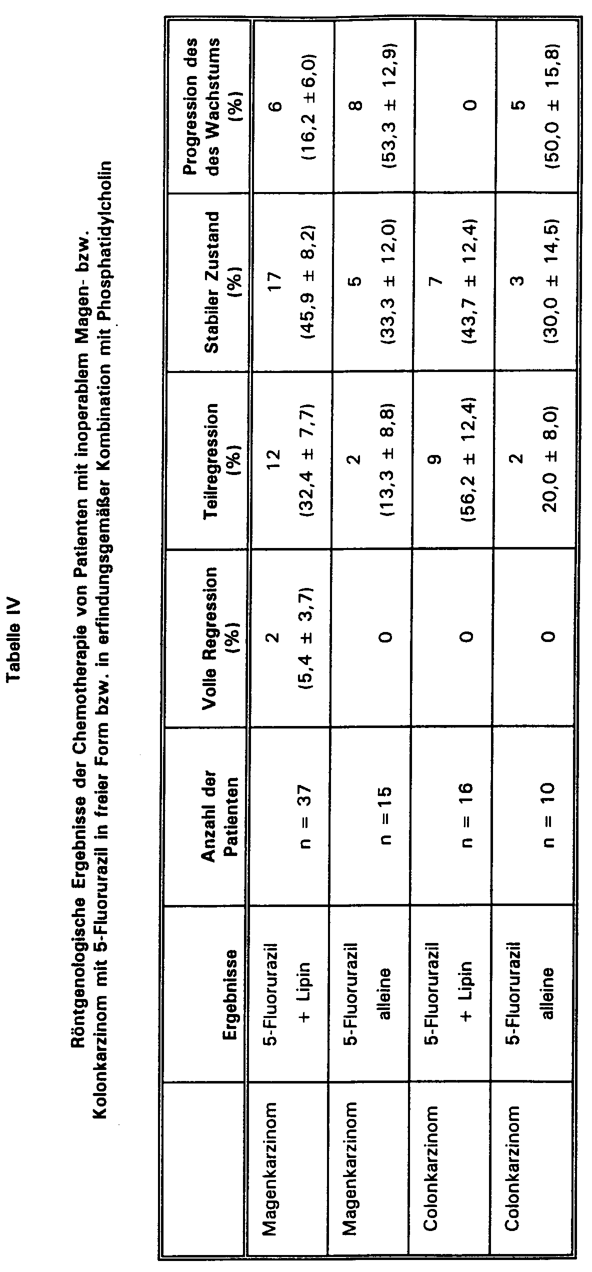

Neben der erhöhten Regressionsrate bzw. der Stabilisierung des Zustandes von Krebspatienten wurde bei der Behandlung mit Zytostatika in Kombination mit der speziellen Liposomenlösung beobachtet, daß die bekannten Nebenwirkungen der Chemotherapeutika erheblich abgeschwächt wurden, wodurch den Patienten in hohem Maße Schmerzen und Unannehmlichkeiten erspart blieben.

### Beispiel 5

### Behandlung von Patienten mit Myokardinfarkten

31 fast ausschließlich männliche Patienten mit akutem, transmuralem Myokardinfarkt wurden einer Standardbehandlung mit Streptokinase, dem Beta Rezeptorblocker Metoprololtartrat und dem Nitrat Nitrendipin behandelt. 20 dieser Patienten (Hauptgruppe) wurden diese Medikamente in Kombination mit einer erfindungsgemäßen Phosphatidylcholinlösung verabreicht. 11 Patienten (Kontrollgruppe) wurden ohne die erfindungsgemäße Lösung behandelt. Alle Patienten erhielten täglich vier Infusionen im Abstand von jeweils sechs Stunden über einen Zeitraum von 5 - 7 Tagen (Hauptgruppe) bzw. 15 - 20 Tagen (Kontrollgruppe). Die Menge an verarbreichter Streptokinase betrug 5 - 10 x 10⁵ Einheiten pro Injektion, je nach Körpergewicht und Schwere des jeweiligen Infarkts. Nitrendipin wurde in einer Menge von 5 mg pro Infusion verabreicht. Der Beta Rezeptorblocker Metoprololtartrat wurde je nach Verträglichkeit in Mengen zwischen 15 und 100 mg pro Infusion verabreicht, wobei die Dosis nach 6 - 7 Tagen in jedem Fall auf höchstens 20 mg herabgesetzt wurde. Der Hauptgruppe wurde 30 Minuten vor jeder Medikamenteninfusion eine bestimmte Mengedererfindungsgemäßen Phosphatidylcholinlösung injiziert. Die erste Dosis der erfindungsgemäßen Liposomenlösung enthielt 30 mg Phosphatidylcholin pro kg Körpergewicht und jede weitere Dosis enthielt 15 mg Phosphatidylcholin pro kg Körpergewicht. Aufgrund der guten Ergebnisse konnte die Behandlung der Hauptgruppe nach 5 - 7 Tagen abgebrochen werden, wogegen die Behandlung der Kontrollgruppe 15 - 20 Tage andauerte und dabei trotzdem schlechtere Ergebnisse erzielt wurden als bei der Hauptgruppe. Die Ergebnisse der Behandlung sind in Tabelle V zusammengefaßt.

**Tabelle V**

| **Der Einfluß der erfindungsgemäßen Phosphatidylcholinlösung auf den Krankheitsverlauf bei der Behandlung von Patienten mit transmuralem Myokardinfarkt.** | | |
|---|---|---|
| Gruppe | Hauptgruppe ( Behandlung mit Lipin) | Kontrollgruppe (Behandlung ohne Lipin) |
| Anzahl der Patienten/Geschlecht/Alter | 20/19 männlich/48-72 Jahre 1 weiblich/65 Jahre | 11/11 männlich/46 - 68 Jahre 0 weiblich |
| Behandlung abgebrochen | 0 | 2(verstroben) |
| Behandlungsdauer | 5 - 7 Tage | 15 - 20 Tage |

| Ergebnisse | | |
|---|---|---|
| positiv | 17 | 1 |
| indifferent | 3 | 8 |
| negativ | 0 | 2 (verstorben) |
| Schmerzsyndrom | 0 | 0 |
| Myokardinfarkt | 0 | 1 |
| Herzrhythmusstörungen | 1 | 5 |
| Maximale Kreatinphosphokinaseaktivität (mcot/l) | 1,56 ± 0,17 | 2,24 ± 0,21 |
| Normalisierungszeitraum der Kreatinphosphokinaseaktivität (Stunden) | 44 ± 4 | 94 ± 8 |
| Normalisierungszeitraum des ST-Segments im ECG (Stunden) | 47 ± 5 | 96 ± 7 |
| Linksseitiges ventrikuläres Ausfallsyndrom | 2 | 6 |
| Häufigkeit muraler Aneurismen | 1 | 3 |

## Patentansprüche

1. Verwendung einer durch Homogenisieren einer Phosphatidylcholinemulsion in Wasser unter erhöhtem Druck, erhöhter Temperatur und Zusatz von Lactose hergestellten Liposomenlösung zur Verstärkung der Wirksamkeit und/oder Verminderung der Toxizität von Arzneimitteln.

2. Verwendung einer Liposomenlösung nach Anspruch 1 in einem Mischungsverhältnis von Phosphatidylcholin zu Arzneimittel von 100 : 1 bis 1 : 1, bevorzugt 60 : 1 bis 20 : 1, besonders bevorzugt 50 : 1 bis 30 : 1, bezogen auf die Trockensubstanzen.

3. Verwendung einer durch Homogenisieren der Phosphatidylcholinemulsion bei einenm Druck von 5,9 x 10⁷ bis 6,3 x 10⁷ Pa, bei einer Temperatur von 40 bis 45 °C und unter Zusatz von Lactose in einer Menge von 1,8 bis 2,2 Gew.% hergestellten Liposomenlösung nach einem der Ansprüche 1 oder 2.

4. Verwendung einer durch Homogenisieren einer Phosphatidylcholinemulsion hergestellten Liposomenlösung nach einem der Ansprüche 1 bis 3, die tierisches, vorzugsweise aus Eidotter gewonnenes, Phosphatidylcholin enthält.

5. Verwendung einer durch Homogenisieren einer Phosphatidylcholinemulsion hergestellten Liposomenlösung nach einem der Ansprüche 1 bis 4 zur Behandlung von Krebserkrankungen.

6. Verwendung einer durch Homogenisieren einer Phosphatidylcholinemulsion hergestellten Liposomenlösung nach einem der Ansprüche 1 bis 5 zur Behandlung von Herzkreislauferkrankungen.
